# EUROPEAN PATENT APPLICATION

(11) **EP 2 149 369 A1**
(43) Date of publication of application: **03.02.2010**
(21) Application number: 08171354.7
(22) Date of filing: 11.12.2008
(51) Int. Cl.: A61K 9/00, A61K 9/14, A61K 38/01, A61K 31/198, A61K 31/205, A61P 3/02

(54) **Instant oral formulations, gelling at room temperature, useful to treat physical wasting and the depressive syndrome associated with cachexia, anorexia, metabolic disorders, endocrine disorders and dysphagia**

(30) Priority: 29.07.2008 IT MI20081405
(71) Applicant: Velleja Research SRL, 20122 Milano (IT)
(72) Inventor: Di Pierro, Francesco, 29010, PONTENURE (PC) (IT)
(74) Representative: Minoja, Fabrizio

(57) **Abstract**

Disclosed are oral formulations containing whey protein, a mixture of tri- and tetrapeptides obtained from hydrolysis of whey protein, and a methyl donor selected from mono-, di- and trimethylglycine and S-adenosyl-methionine.

## Description

The present invention relates to instant oral formulations gelling at room temperature, comprising a mixture of tri- and tetrapeptides obtained from hydrolysis of whey proteins, and a methyl donor selected from mono-, di- and trimethylglycine.

### Prior art

Cachexia is a serious physical wasting syndrome, characterised by progressive deterioration of the metabolic functions, and usually associated with anorexia nervosa, endocrine disorders, malignant tumours and dementia.

Neoplastic cachexia in particular is a seriously debilitating syndrome characterised by weight loss, anorexia, asthenia, loss of muscle mass, depletion of the adipose deposits and profound metabolic alterations.

It is characterised by a major weight loss (which may exceed 10 kg), and is present in at least 50% of cancer patients. As well as adversely affecting the quality of life of patients and their families, cachexia also reduces the response to cancer treatments and significantly increases mortality. The main and most serious clinical characteristic of neoplastic cachexia, namely progressive loss of muscle mass, is considered by many authors to be irreversible by common nutritional, metabolic and pharmacological treatments. Depression, another typical characteristic which very often accompanies physical wasting, leads to loss of appetite and aggravates the symptoms of the disorder.

As a direct result of their illness and depressive syndrome, cachexia patients have a general protein turnover which is much faster than those of healthy volunteers due to the production of cytokines such as Tumour Necrosis Factor alpha (TNF-alpha) and interleukin 1 (IL-1), which stimulate proteolysis and catabolism.

Many drugs have been used to treat this phenomenon, with low efficacy. They include cyproheptadine and anabolic steroids, which have serious side effects.

The body and muscle wasting typical of cachexia is also characteristic of anorexia (often called anorexia "nervosa"). Two forms of anorexia nervosa can be distinguished: restrictive anorexia, a form in which weight loss is caused by fasting and intense physical activity, and anorexia with bulimia, a form in which the patient acts in ways which, together with fasting, serve to reduce the body weight (abuse of laxatives and/or diuretics, vomiting). Physical wasting and depressive syndrome, first described for cachexia and now for anorexia, can also be observed as the direct consequence of other disorders such as dysphagia, primary metabolic syndromes or metabolic syndromes secondary to other disorders and endocrine and/or neuroendocrine disorders.

Dysphagia, in particular, is a dysfunction of the digestive apparatus consisting of difficulty in swallowing. It nearly always occurs as a result of other obstructive or motor disorders, such as the presence of tumours or achalasia. It usually only relates to solid foods, which the patient finds very difficult to swallow, and much more rarely to semi-liquid or liquid foods. Swallowing difficulty leads to major weight losses, especially as regards the muscle component, as in all cases of food deprivation, similarly to the cachectic symptoms of physical wasting associated with depressive syndrome, mood disorders and anxiety. The medical profession is still debating whether dysphagia has its own symptoms, or the symptoms observed (coughing, recurrent respiratory infections, regurgitation, and difficulty in swallowing with the risk of suffocation) are always solely correlated with its etiology. Dysphagia may have obstructive or motor causes. The first category includes the presence of foreign bodies, stenosis, tumours, inflammations and cervical spondylitis. The second includes polymyositis, amyotrophic lateral sclerosis, achalasia and scleroderma.

Much of the physical wasting resulting from cachexia, anorexia, dysphagia and metabolic and/or endocrine syndrome is associated with an excessive catabolic event affecting proteins, which is not counterbalanced by a sufficient intake and assimilation capacity. A low protein intake consequently reduces muscle mass and tone, while only a correct protein intake followed by effective assimilation is eutrophic for the tissues and can counteract physical wasting. The most important parameter of a protein intake designed to counteract physical wasting is not its amount; on the contrary, quality is far more critical to the metabolic destiny of proteins.

As there are many potential sources of protein (whey protein, caseinates, milk protein isolates, soya protein, egg protein and hydrolyzed wheat protein), some authors (Block and Mitchell, 1946) proposed the aminoacid combination of egg protein as the reference standard to evaluate the quality of protein for use in human patients. That standard was modified in later years, and the introduction of new standards has changed the analysis of protein quality to some extent. The following methods have been recognised and are currently used to evaluate protein quality:
1) protein efficiency ratio (PER): this parameter is generally measured experimentally under standard nutritional conditions. The PER is defined as the weight increase (g) per gram of protein intake. The PER value of milk protein is 3.1, casein 2.0, whey protein 3.6, soya protein 2.1 and wheat protein 1.5;
2) digestibility (D): true digestibility is the ratio between the protein nitrogen absorbed and the amount of protein nitrogen ingested. Said ratio is then corrected for the metabolic losses of nitrogen with the faeces. The true digestibility values of proteins are 96% for wheat flour, 95% for milk protein, 95% for soya protein isolate and 86% for soya flour;
3) biological value (BV): this term indicates the quality of nitrogen absorbed from a protein which was retained for maintenance and/or growth purposes. The reference protein is egg protein, the BV of which is 100. This is followed by milk protein 91, casein 77, whey 104, soya 74 and corn 54;
4) Net Protein Use (NPU): this term relates to the ratio between the nitrogen intake and the nitrogen retained; NPU can be calculated by multiplying the BV by the D of a protein;
5) Net Nitrogen Use (NNU): this parameter measures the amount of nitrogen actually assimilated by the subject;
6) Protein Digestibility Corrected Amino Acid Score (PDCAAS), currently the most advanced protein quality measurement index, was developed by the WHO. Its maximum value is 1.0. Every protein with a value of 1.0 is therefore considered complete for humans.

On the basis of said parameters, there are two excellent protein sources: egg and whey. Egg white, like yolk, has an optimum protein profile and can be considered a complete, high-quality source of protein. Eggs are easily digested by the human body, but the absorption time of this protein is rather long, and absorption is never total.

Whey proteins are undoubtedly the best protein source. These proteins, when subjected to particular treatments (hydrolysis), have a very high degree of oral bioavailability and, above all, supply peptides able to influence the levels of cytokines and lymphokines, including TNF-alpha and IL-1, directly involved in the cachexia and physical wasting processes. Whey also contains tetrapeptides which act as natural opiates with anti-depressant and analgesic activity, and glutathione-like antioxidant tripeptides (with antioxidant activity). Finally, whey is very rich in branched-chain aminoacids (L-isoleucine, L-valine and L-leucine) whose absence often limits the anabolic phenomena required to counteract physical wasting.

### Description of the invention

It has now been found that a combination of whey proteins with a hydrolysate of the same proteins and a methyl donor compound is particularly effective in treating the physical wasting typical of cachexia, and also helps to reduce the depressive symptoms and anxiety typical of these disorders.

The formulations according to the invention supply the maximum protein intake with an excellent degree of oral bioavailability in terms of total absorption, absorption rate (in relation to stomach voiding time), post-prandial oxidation risk, and use in protein synthesis.

The invention therefore provides oral formulations containing whey protein, a mixture of tri- and tetra-peptides obtained from hydrolysis of whey protein, and a methyl donor chosen from mono-, di- and tri-methylglycine, preferably dimethylglycine.

The formulations according to the invention are preferably in a solid oral form which gels instantly at room temperature, and contain 5 to 50 g of di-, tri- and tetrapeptides from whey protein, 5 to 50 g of whey protein and 10 to 1500 mg of methyl donor per unit dose, and preferably 100 mg of dimethylglycine per unit dose.

Examples of suitable gelling agents are hydrolyzed guar gum, carrageenan, colloidal polymers, and partly hydrolyzed glucomannan.

The formulations may also contain one or more of the following ingredients:
- ubidecarenone, glutathione, cystine, cysteine, methionine, alphalipoic acid, S-adenosylmethionine, N-acetylcysteine;
- extracts of *Vitis vinifera*, bilberry, tea and milk thistle;
- vitamin C, vitamin E, carotenoids and vitamin B;
- Mg, K, Se, Cu and Fe salts;
- taurine, carnitine and carnosine;
- creatine and phosphocreatine.

The hydrolysis of whey protein, conducted as far as the formation of tri- and tetra-peptides, produces peptone mixtures with a low molecular weight whose PDCAAS is very high because the absorption percentage, measured in tests on healthy volunteers, is among the highest known (exceeding 95%), with a very high absorption rate (the C ₘₐₓ can already be measured after 60 min), a very low degree of post-prandial oxidation (less than 0.5 micromol/Kg⁻¹/min⁻¹), and a 75% increase in post-prandial protein synthesis, measured after 420 min. Said hydrolysates are known and available on the market, for example from DMV and Ingredia.

Other protein hydrolysates, in which hydrolysis does not continue until the appearance of tri- and tetra peptides, have worse properties: measured AUC being equal, they present a low absorption rate (which induces protein catabolism and demolition of the lean mass), high post-prandial oxidation (for example, non-hydrolyzed caseinates reach 1.5 micromol/Kg⁻¹/min⁻¹) and reduced new protein synthesis (70% for weakly hydrolyzed serum proteins, ie. without the appearance of tri- and tetra peptides; approx. 60% for wheat protein, and approx. 30% for caseinates). This hydrolyzed protein profile is not only responsible for the eutrophic effect on the muscle mass but also for promoting a reduction in the genesis of cachexia associated with the release of TNF-alpha and IL-1, which are often jointly responsible for that syndrome. Moreover, the plasma absorption of tri- and tetrapeptides causes an evident anti-depressant and anti-anxiety effect.

The presence of non-hydrolyzed protein considerably improves the organoleptic characteristics of the formulations and produces a second plasma absorption curve for protein derivatives with low molecular weight deriving from digestion of said protein. The combination of equivalent doses of protein hydrolyzed from serum and non-hydrolyzed protein produces a first plasma aminoacid absorption curve which is associated with the hydrolyzed protein portion, and a second curve deriving from the non-hydrolyzed protein portion.

The formulations according to the invention also possess advantageous anti-depressant and mood up-modulating properties due to the addition of monomethylglycine, dimethylglycine and/or trimethylglycine, which help to improve the profile of the biogenic amines involved. In the depression or anxiety syndromes typical of cachectic states with various etiologies, said amines may be down-modulated due to excessive subtraction from the intersynaptic environment, or their specific receptors may be down-modulated.

The combinations according to the invention are useful not only to counteract muscle wasting, whether or not it is associated with a depressive syndrome, but also to correct poor eating habits in obese patients and/or type II diabetics and to improve sporting performance in healthy subjects.

The specific therapeutic indications of the formulations according to the invention include:
1) neoplastic and non-neoplastic cachexia;
2) physical wasting associated with anorexia;
3) physical wasting associated with a primary or secondary metabolic syndrome;
4) physical wasting with an endocrine or neuroendocrine syndrome;
5) treatment of type II diabetes;
6) treatment of bulimia;
7) treatment of obesity (including at paediatric age);
8) treatment of dysphagia.

The invention is illustrated in detail in the following examples.

### EXAMPLES

### EXAMPLE 1

| Ingredient | Amount |
|---|---|
| Non-hydrolyzed milk protein (85%) | 12.50 g |
| Hydrolyzed whey protein | 12.50 g |
| Fructose | 10.80 g |
| Lecithinated low-fat cocoa | 6.00 g |
| Guar gum | 16.00 g |
| Carrageenan | 9.00 g |
| Flavourings | 300 mg |
| Monobasic potassium phosphate | 200 mg |
| Dimethylglycine | 100 mg |
| Silicon dioxide | 100 mg |
| Magnesium oxide | 74.7 mg |
| Sucralose | 10 mg |
| Chromium chloride | 0.06 mg |

### EXAMPLE 2

| Ingredient | Amount |
|---|---|
| Non-hydrolyzed milk protein (85%) | 12.50 g |
| Hydrolyzed whey protein | 12.50 g |
| Fructose | 10.80 g |
| Lecithinated low-fat cocoa | 6.00 g |
| Guar gum | 16.00 g |
| Carrageenan | 9.00 g |
| Flavourings | 300 mg |
| Monobasic potassium phosphate | 200 mg |
| Dimethylglycine | 100 mg |
| SAMe | 200 mg |
| Silicon dioxide | 100 mg |
| Magnesium oxide | 74.7 mg |
| Sucralose | 10 mg |
| Chromium chloride | 0.06 mg |

### EXAMPLE 3

| Ingredient | Amount |
|---|---|
| Non-hydrolyzed milk protein (85%) | 12.50 g |
| Hydrolyzed whey protein | 12.50 g |
| Fructose | 10.80 g |
| Strawberry flavouring | 6.00 g |
| Guar gum | 16.00 g |
| Carrageenan | 9.00 g |
| Flavourings | 300 mg |
| Monobasic potassium phosphate | 200 mg |
| Dimethylglycine | 100 mg |
| SAMe | 200 mg |
| GSH | 30 mg |
| Silicon dioxide | 100 mg |
| Magnesium oxide | 74.7 mg |
| Sucralose | 10 mg |
| Chromium chloride | 0.06 mg |

### EXAMPLE 4

| Ingredient | Amount |
|---|---|
| Non-hydrolyzed milk protein (85%) | 12.50 g |
| Hydrolyzed whey protein | 12.50 g |
| Fructose | 10.80 g |
| Strawberry flavouring | 6.00 g |
| Guar gum | 16.00 g |
| Carrageenan | 9.00 g |
| Flavourings | 300 mg |
| Monobasic potassium phosphate | 200 mg |
| Dimethylglycine | 100 mg |
| Lycopene | 6 mg |
| OPC (Vitis vinifera) | 100 mg |
| GSH | 30 mg |
| Silicon dioxide | 100 mg |
| Magnesium oxide | 74.7 mg |
| Acesulfame K | 20 mg |
| Aspartame | 20 mg |
| Chromium chloride | 0.06 mg |

### EXAMPLE 5

| Ingredient | Amount |
|---|---|
| Non-hydrolyzed milk protein (85%) | 12.50 g |
| Hydrolyzed whey protein | 12.50 g |
| Fructose | 10.80 g |
| Strawberry flavouring | 6.00 g |
| Guar gum | 16.00 g |
| Carrageenan | 9.00 g |
| Flavourings | 300 mg |
| Monobasic potassium phosphate | 200 mg |
| Dimethylglycine | 100 mg |
| SAMe | 200 mg |
| Creatine | 250 mg |
| Silicon dioxide | 100 mg |
| Magnesium oxide | 74.7 mg |
| Sucralose | 10 mg |
| Chromium chloride | 0.06 mg |

### EXAMPLE 6

| Ingredient | Amount |
|---|---|
| Non-hydrolyzed milk protein (85%) | 12.50 g |
| Hydrolyzed whey protein | 12.50 g |
| Fructose | 10.80 g |
| Strawberry flavouring | 6.00 g |
| Guar gum | 16.00 g |
| Carrageenan | 9.00 g |
| Flavourings | 300 mg |
| Monobasic potassium phosphate | 200 mg |
| Dimethylglycine | 100 mg |
| SAMe | 200 mg |
| Creatine | 250 mg |
| Taurine | 100 mg |
| Silicon dioxide | 100 mg |
| Magnesium oxide | 74.7 mg |
| Sucralose | 10 mg |
| Chromium chloride | 0.06 mg |

### EXAMPLE 7

| Ingredient | Amount |
|---|---|
| Non-hydrolyzed milk protein (85%) | 12.50 g |
| Hydrolyzed whey protein | 12.50 g |
| Fructose | 10.80 g |
| Vanilla flavouring | 1.00 g |
| Guar gum | 16.00 g |
| Carrageenan | 9.00 g |
| Flavourings | 300 mg |
| Monobasic potassium phosphate | 200 mg |
| Dimethylglycine | 100 mg |
| SAMe | 200 mg |
| Creatine | 250 mg |
| Taurine | 100 mg |
| CLA | 600 mg |
| Silicon dioxide | 100 mg |
| Magnesium oxide | 74.7 mg |
| Sucralose | 10 mg |
| Chromium chloride | 0.06 mg |

## Claims

1. Oral formulations comprising whey proteins, a mixture of tri- and tetrapeptides obtained by hydrolysis of whey proteins and a methyl donor selected from mono-, di-, trimethylglycine and S-adenosyl-methionine.

2. Oral formulations as claimed in claim 1, wherein the methyl donor is dimethylglycine.

3. Formulations as claimed in claim 1 or 2 comprising 5 to 50 g of di-, tri- and tetrapeptides from whey proteins, 5 to 50 g of whey proteins and 10 to 1500 mg of methyl donor per unit dose.

4. Formulations as claimed in claim 3 containing 100 mg of dimethylglycine per unit dose.

5. Formulations as claimed in any one of claims 1 to 4, further comprising one or more of the following components:
- ubidecarenone, glutathione, cystine, cysteine, methionine, alphalipoic acid, N-acetylcysteine, S-adenosyl-methionine;
- extracts of *Vitis vinifera*, bilberry, tea, milk thistle;
- vitamin C, vitamin E, carotenoids, vitamins B;
- Mg, K, Se, Cu, Fe salts;
- taurine, carnitine, carnosine;
- creatine, phosphocreatine.

6. Formulations as claimed in any one of claims 1 to 5 comprising a gelling agent selected from hydrolyzed guar gum, carrageenin, colloidal polymers, partially hydrolyzed glucomannan.

7. The use of tri- and tetrapeptides obtained by hydrolysis of whey proteins in combination with whey proteins and with a methyl donor selected from mono-, di- and trimethylglycine, for the preparation of compositions for the treatment of cachexia associated with even mild depressive syndrome.
